# EUROPEAN PATENT APPLICATION

(11) **EP 4 410 219 A1**
(43) Date of publication of application: **07.08.2024**
(21) Application number: 22875540.1
(22) Date of filing: 11.07.2022
(51) Int. Cl.: A61B 8/12, A61B 1/00

(54) **ULTRASONIC ENDOSCOPE SYSTEM AND OPERATING METHOD FOR ULTRASONIC ENDOSCOPE SYSTEM**

(30) Priority: 30.09.2021 JP 2021160406
(71) Applicant: FUJIFILM Corporation, Tokyo 106-8620 (JP)
(72) Inventor: KARASAWA, Hiroyuki, Ashigarakami-gun, Kanagawa 258-8538 (JP)
(74) Representative: Hughes, Andrea Michelle
(86) International application number: PCT/JP2022/027270
(87) International publication number: WO 2023/053662

(57) **Abstract**

An ultrasonic endoscope system includes: an endoscope operation part that is operated by a user; an endoscope insertion part that is connected to the endoscope operation part and is to be inserted into a subject; an ultrasonic transducer array that is disposed at a distal end of the endoscope insertion part; an endoscope image processor section that generates an endoscope image based on an image signal acquired optically by the endoscope insertion part; an ultrasound image processor section that is housed in the endoscope operation part and generates ultrasound image information data by performing signal processing on a reception signal output from the ultrasonic transducer array; and a display unit that displays an ultrasound image based on the ultrasound image information data and the endoscope image.

## Description

### BACKGROUND OF THE INVENTION

### 1. Field of the Invention

The present invention relates to an ultrasonic endoscope system and a method of operating the ultrasonic endoscope system, and particularly relates to an ultrasonic endoscope system that observe a state of an observation target part inside a body of a subject using ultrasonic waves, and a method of operating the ultrasonic endoscope system.

### 2. Description of the Related Art

An ultrasonic endoscope system inserts an endoscope insertion part having a distal end at which an endoscope observation part and an ultrasonic observation part are disposed into a digestive tract of a subject, and captures an endoscope image of an inside of the digestive tract and an ultrasound image of an observation target part located outside a digestive tract wall.

In the ultrasonic endoscope system, an observation target adjacent part inside the digestive tract is irradiated with illumination light from an illumination part in the endoscope observation part, reflected light of the illumination light is received by an imaging part in the endoscope observation part, and an endoscope image is generated from a reception signal of the reflected light. In addition, an ultrasonic transducer array disposed in the ultrasonic observation part is driven to transmit and receive an ultrasonic wave to and from the observation target part located outside the digestive tract wall, and an ultrasound image is generated based on a reception signal output from the ultrasonic transducer array.

This type of ultrasonic endoscope system is disclosed in, for example, JP2021-13453A and JP5815160B.

In the ultrasonic endoscope system disclosed in JP2021-13453A, a light source device, an endoscope image processor device, and an ultrasound image processor device are connected to an ultrasonic endoscope. The reception signal received by the imaging part of the endoscope observation part is transmitted to the endoscope image processor device, and an endoscope image is generated in the endoscope image processor device. The reception signal output from the ultrasonic transducer array of the ultrasonic observation part is transmitted to the ultrasound image processor device, and an ultrasound image is generated in the ultrasound image processor device.

In addition, also in the ultrasonic endoscope system disclosed in JP5815160B, the reception signal received by the imaging part of the endoscope observation part is transmitted to the endoscope image processor device connected to the ultrasonic endoscope, and an endoscope image is generated in the endoscope image processor device. Note that the ultrasonic endoscope includes a wireless communication unit, and the reception signal acquired by the ultrasonic transducer array is wirelessly transmitted to an ultrasonic observation device disposed outside the ultrasonic endoscope by the wireless communication unit, and an ultrasound image is generated in the ultrasonic observation device.

### SUMMARY OF THE INVENTION

However, in the ultrasonic endoscope systems disclosed in JP2021-13453A and JP5815160B, an endoscope image processor device that generates an endoscope image and an ultrasound image processor device or an ultrasonic observation device that generates an ultrasound image need to be provided outside the ultrasonic endoscope, and there is a problem that a configuration of the ultrasonic endoscope system is complicated and large.

The present invention has been made to solve such a problem in the related art, and an object of the present invention is to provide an ultrasonic endoscope system having a simple configuration and a small size, and a method of operating the ultrasonic endoscope system.

According to the following configuration, the above object can be achieved.
[1] An ultrasonic endoscope system comprising: an endoscope operation part that is operated by a user; an endoscope insertion part that is connected to the endoscope operation part and is to be inserted into a subject; an ultrasonic transducer array that is disposed at a distal end of the endoscope insertion part; an endoscope image processor section that generates an endoscope image based on an image signal acquired optically by the endoscope insertion part; an ultrasound image processor section that is housed in the endoscope operation part and generates ultrasound image information data by performing signal processing on a reception signal output from the ultrasonic transducer array; and a display unit that displays an ultrasound image based on the ultrasound image information data and the endoscope image.
[2] The ultrasonic endoscope system according to [1], in which the ultrasound image processor section includes an ultrasound image information data transmission unit that transmits the ultrasound image information data to the endoscope image processor section.
[3] The ultrasonic endoscope system according to [2], in which the ultrasound image information data transmission unit transmits the ultrasound image information data to the endoscope image processor section through serial communication.
[4] The ultrasonic endoscope system according to [2] or [3], in which the ultrasound image information data transmission unit wirelessly transmits the ultrasound image information data to the endoscope image processor section.
[5] The ultrasonic endoscope system according to [4], further comprising: an input device that is connected to the endoscope image processor section and is used by the user to perform an input operation.
[6] The ultrasonic endoscope system according to [5], further comprising: an operation console on which the input device is disposed, in which the operation console has a wireless communication circuit that receives the ultrasound image information data wirelessly transmitted from the ultrasound image information data transmission unit and sends the ultrasound image information data to the endoscope image processor section.
[7] The ultrasonic endoscope system according to any one of [2] to [6], in which the endoscope image processor section includes an image combining unit that generates a composite image in which the ultrasound image is combined with the endoscope image.
[8] The ultrasonic endoscope system according to [7], in which the display unit consists of a monitor for displaying the composite image.
[9] The ultrasonic endoscope system according to [1], in which the display unit includes a first monitor for displaying the endoscope image and a second monitor for displaying the ultrasound image.
[10] The ultrasonic endoscope system according to any one of [1] to [9], in which the ultrasound image information data is intermediate data obtained by subjecting a sound ray signal generated by performing the signal processing on the reception signal to attenuation correction according to a depth of a position where an ultrasonic wave is reflected and detection processing.
[11] The ultrasonic endoscope system according to any one of [1] to [9], in which the ultrasound image information data is ultrasound image data obtained by subjecting a sound ray signal generated by performing the signal processing on the reception signal to attenuation correction according to a depth of a position where an ultrasonic wave is reflected and detection processing and converting the sound ray signal according to a predetermined image display method.
[12] The ultrasonic endoscope system according to any one of [1] to [11], in which the endoscope image processor section has a power supply circuit that generates an internal power supply voltage, and the ultrasound image processor section drives the ultrasonic transducer array using the internal power supply voltage generated by the power supply circuit and generates the ultrasound image information data.
[13] The ultrasonic endoscope system according to any one of [1] to [12], in which the endoscope operation part has an operation member and a control substrate for controlling the operation member, and the ultrasound image processor section is mounted on the control substrate.
[14] The ultrasonic endoscope system according to any one of [1] to [13], in which the endoscope operation part has a connection metal portion to be connected to the endoscope insertion part, a heat transfer member that connects the ultrasound image processor section and the connection metal portion is provided, and heat generated by the ultrasound image processor section is transmitted to the connection metal portion via the heat transfer member.
[15] A method of operating an ultrasonic endoscope system, the method comprising: imaging an inside of a subject with an endoscope insertion part connected to an endoscope operation part; generating an endoscope image by an endoscope image processor section based on an image signal acquired optically by the endoscope insertion part; transmitting and receiving an ultrasonic wave by using an ultrasonic transducer array disposed at a distal end of the endoscope insertion part; generating ultrasound image information data for the inside of the subject by performing signal processing on a reception signal output from the ultrasonic transducer array by an ultrasound image processor section housed in the endoscope operation part; and displaying an ultrasound image based on the ultrasound image information data and the endoscope image on a display unit.

According to the present invention, since the ultrasound image processor section that is housed in the endoscope operation part and generates the ultrasound image information data by performing the signal processing on the reception signal output from the ultrasonic transducer array is provided, it is possible to simplify and reduce the size of the configuration of the ultrasonic endoscope system.

### BRIEF DESCRIPTION OF THE DRAWINGS

Fig. 1 is a diagram schematically showing a configuration of an ultrasonic endoscope system according to Embodiment 1 of the present invention.
Fig. 2 is a diagram showing a distal end of an endoscope insertion part and a periphery thereof.
Fig. 3 is a cross-sectional view taken along the line I-I of Fig. 2.
Fig. 4 is a block diagram showing the configuration of the ultrasonic endoscope system according to Embodiment 1.
Fig. 5 is a block diagram showing an internal configuration of a reception circuit in Embodiment 1.
Fig. 6 is a diagram schematically showing a composite image generated by an image combining unit in Embodiment 1.
Fig. 7 is a diagram schematically showing another composite image generated by the image combining unit in Embodiment 1.
Fig. 8 is a diagram showing an ultrasound image processor section that is housed in an endoscope operation part in Embodiment 1 and is mounted on a circuit board.
Fig. 9 is a partial cross-sectional view showing a connection portion between the endoscope operation part and the endoscope insertion part in Embodiment 1.
Fig. 10 is a flowchart showing an operation of the ultrasonic endoscope system according to Embodiment 1.
Fig. 11 is a block diagram showing a configuration of an ultrasonic endoscope system according to Embodiment 2.
Fig. 12 is a block diagram showing a configuration of an ultrasonic endoscope system according to Embodiment 3.
Fig. 13 is a block diagram showing a configuration of an ultrasonic endoscope system according to Embodiment 4.
Fig. 14 is a block diagram showing a configuration of an ultrasonic endoscope system according to Embodiment 5.
Fig. 15 is a block diagram showing a configuration of an ultrasonic endoscope system according to Embodiment 5.
Fig. 16 is a block diagram showing a configuration of an ultrasonic endoscope system according to Embodiment 5.
Fig. 17 is a diagram showing an ultrasound image processor section that is housed in an endoscope operation part of an ultrasonic endoscope system according to Embodiment 6 and is mounted on a control substrate.

### DESCRIPTION OF THE PREFERRED EMBODIMENTS

Hereinafter, embodiments of the present invention will be described with reference to the accompanying drawings.

Description of configuration requirements described below may be made based on a typical embodiment of the present invention, but the present invention is not limited to such an embodiment.

In the present specification, the numerical range represented by "to" means a range including numerical values denoted before and after "to" as a lower limit value and an upper limit value.

In the present specification, the terms "same" and "identical" include an error range generally allowed in the technical field.

### Embodiment 1

### [Outline of Ultrasonic Endoscope System 1]

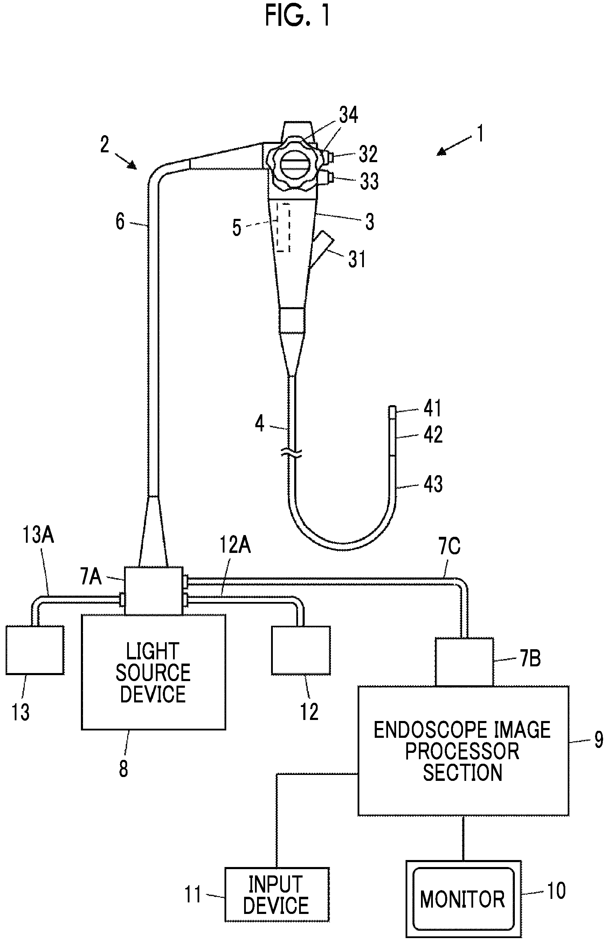

The outline of the ultrasonic endoscope system 1 according to Embodiment 1 will be described with reference to Fig. 1.

The ultrasonic endoscope system 1 acquires an ultrasound image and an endoscope image, and is used for observing a state of an observation target part in a body of a patient who is a subject. Here, the observation target part is a part that is difficult to be examined from a body surface side of the patient, and is, for example, pancreas or gallbladder. With the use of the ultrasonic endoscope system 1, it is possible to diagnose a state of the observation target part and the presence or absence of an abnormality by way of digestive tracts, such as esophagus, stomach, duodenum, small intestine, and large intestine, which are body cavities of the patient.

The ultrasonic endoscope system 1 comprises an ultrasonic endoscope 2. The ultrasonic endoscope 2 has an endoscope operation part 3 that is operated by a user such as a doctor or a technician, and an endoscope insertion part 4 that is connected to the endoscope operation part 3 and is to be inserted into the body cavity of the patient, in which an ultrasound image processor section 5 is housed inside the endoscope operation part 3.

In addition, a light source device 8 is connected to the endoscope operation part 3 via a universal cable 6 and a universal connector 7A. An endoscope image processor section 9 is connected to the universal connector 7A via a connection cable 7C and an endoscope connector 7B, and a monitor (display unit) 10 and an input device 11 are connected to the endoscope image processor section 9. In addition, a water supply tank 12 and a suction pump 13 are connected to the universal connector 7A.

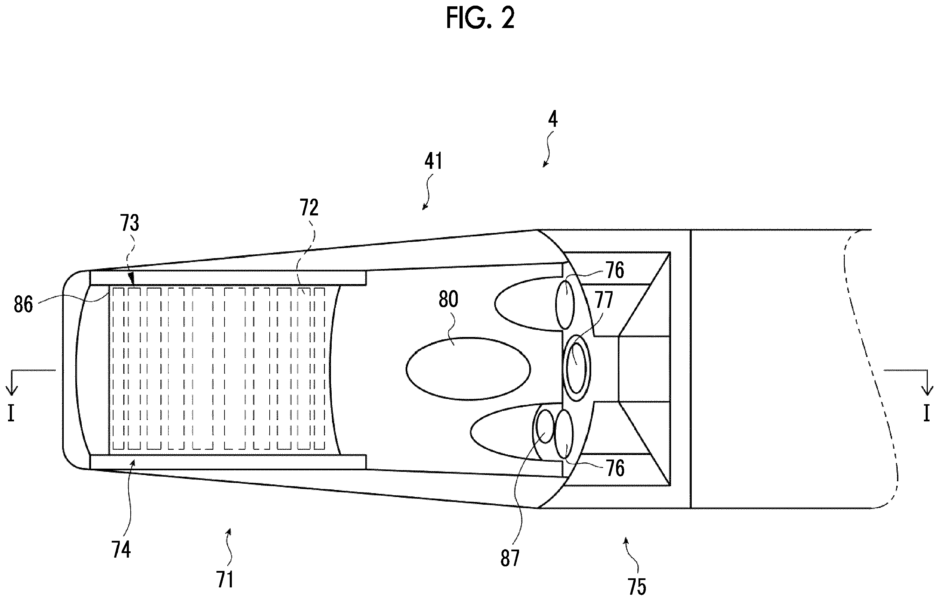

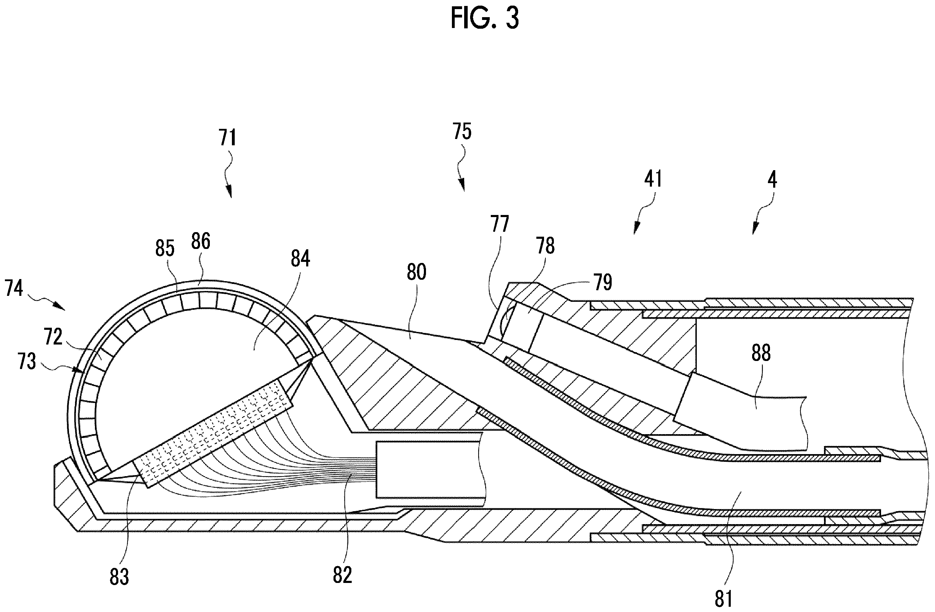

As shown in Figs. 2 and 3, in a distal end portion 41 of the endoscope insertion part 4 of the ultrasonic endoscope 2, an ultrasonic transducer unit 74 having an ultrasonic transducer array 73 in which a plurality of ultrasonic transducers 72 are arranged is disposed as an ultrasonic observation part 71, and an illumination part including an illumination window 76 and the like and an imaging part including an observation window 77, an objective lens 78, a charge coupled device (CCD) 79, and the like are disposed as an endoscope observation part 75. The user acquires an endoscope image and an ultrasound image by a function of the ultrasonic endoscope 2.

Here, the term "endoscope image" refers to an image obtained based on an image signal optically acquired by imaging an interior wall of the body cavity of the patient by an optical method. In addition, the term "ultrasound image" refers to an image obtained by receiving reflected waves (echoes) of ultrasonic waves transmitted from the inside of the body cavity of the patient toward the observation target part and imaging reception signals.

The ultrasonic endoscope 2 will be described in detail in a below section.

The endoscope image processor section 9 acquires an image signal of an observation target adjacent part imaged by the CCD 79 of the ultrasonic endoscope 2, and generates an endoscope image by performing predetermined image processing on the acquired image signal. In other words, the endoscope image processor section 9 makes the imaging part disposed in the distal end portion 41 of the endoscope insertion part 4 of the ultrasonic endoscope 2 receive reflected light of the illumination light emitted from the illumination part also disposed at the distal end portion 41 of the endoscope insertion part 4 of the ultrasonic endoscope 2, and generates an endoscope image for diagnosis (hereinafter, simply referred to as an endoscope image) from an imaging signal of the reflected light.

Here, the term "observation target adjacent part" refers to a portion of the interior wall of the body cavity of the patient that is located adjacent to the observation target part.

The endoscope image processor section 9 will be described in detail in a below section.

The ultrasound image processor section 5 housed in the endoscope operation part 3 controls the ultrasonic transducer unit 74 of the ultrasonic endoscope 2 to transmit ultrasonic waves, and generates an ultrasound image by imaging a reception signal in a case in which the ultrasonic transducer unit 74 receives reflected waves (echoes) of the transmitted ultrasonic waves. In other words, the ultrasound image processor section 5 drives the ultrasonic transducer array 73 included in the ultrasonic transducer unit 74 to transmit and receive the ultrasonic waves, and generates an ultrasound image for diagnosis (hereinafter, simply referred to as an ultrasound image) from a reception signal of the ultrasonic waves.

The ultrasound image processor section 5 will be described in detail in a below section.

The light source device 8 irradiates the observation target adjacent part with white light composed of three primary color light of red light, green light, and blue light or light having a specific wavelength in imaging the observation target adjacent part using the ultrasonic endoscope 2. The light emitted from the light source device 8 propagates through the ultrasonic endoscope 2 through a light guide (not shown) included in the universal cable 6 and is emitted from the illumination window 76 of the endoscope observation part 75. With this, the observation target adjacent part is illuminated with the light from the light source device 8.

The monitor 10 constitutes a display unit that displays the ultrasound image generated by the ultrasound image processor section 5 and the endoscope image generated by the endoscope image processor section 9, and includes, for example, a display device such as a liquid crystal display (LCD) or an organic electroluminescence display (organic EL display).

The input device 11 is a device that is used by the user to perform an input operation, and is used for inputting information necessary for ultrasound diagnosis and for providing a start instruction of the ultrasound diagnosis with respect to the ultrasound image processor section 5 via the endoscope image processor section 9. The input device 11 is configured of, for example, a keyboard, a mouse, a trackball, a touchpad, and a touch panel.

Specifically, the user inputs examination information (for example, examination order information including date, an order number, and the like and patient information including a patient identification number, a patient name, and the like) through the input device 11 in a stage before the ultrasound diagnosis is started. After the completion of the input of the examination information, in a case in which the user provides an instruction to start the ultrasound diagnosis through the input device 11, the ultrasound image processor section 5 generates the ultrasound image such that the ultrasound diagnosis is executed based on the input examination information.

In addition, the user can set various control parameters through the input device 11 in executing the ultrasound diagnosis. As the control parameters, for example, a selection result of a live mode and a freeze mode, a set value of a display depth (depth), and a selection result of an ultrasound image generation mode are used.

Here, the term "live mode" refers to a mode in which ultrasound images (video images) obtained at a predetermined frame rate are displayed successively (displayed in real time). The term "freeze mode" refers to a mode in which an image (static image) of one frame of ultrasound images (video images) generated in the past is read from a cine memory (not shown) and displayed.

### [Configuration of Ultrasonic Endoscope 2]

Next, a configuration of the ultrasonic endoscope 2 will be described with reference to Figs. 1 to 3. Fig. 2 is an enlarged view of the distal end portion 41 of the endoscope insertion part 4 of the ultrasonic endoscope 2 and a periphery of the distal end portion 41. Fig. 3 is a cross-sectional view in a case in which the distal end portion 41 of the endoscope insertion part 4 of the ultrasonic endoscope 2 is cut along the line I-I of Fig. 2.

The ultrasonic endoscope 2 has the endoscope insertion part 4 and the endoscope operation part 3 as described above. As shown in Fig. 1, the endoscope insertion part 4 comprises the distal end portion 41, a bendable portion 42, and a soft portion 43 in order from a distal end side (free end side). As shown in Fig. 2, the ultrasonic observation part 71 and the endoscope observation part 75 are disposed in the distal end portion 41. As shown in Fig. 3, the ultrasonic transducer unit 74 comprising the plurality of ultrasonic transducers 72 is disposed in the ultrasonic observation part 71.

In addition, as shown in Fig. 2, a treatment tool outlet port 80 is provided in the distal end portion 41. The treatment tool outlet port 80 serves as an outlet of a treatment tool (not shown) such as forceps, a puncture needle, or a high-frequency scalpel. In addition, the treatment tool outlet port 80 also serves as a suction port in sucking aspirates such as blood or internal filth.

The bendable portion 42 is a portion consecutively installed on a base end side (a side opposite to a side on which the ultrasonic transducer unit 74 is provided) with respect to the distal end portion 41, and is freely bent. The soft portion 43 is an elongated extending portion that connects the bendable portion 42 and the endoscope operation part 3, and has flexibility.

A plurality of pipe lines for air/water supply and a plurality of pipe lines for suction are formed inside each of the endoscope insertion part 4 and the endoscope operation part 3. Furthermore, a treatment tool channel 81 of which one end communicates with the treatment tool outlet port 80 is formed inside each of the endoscope insertion part 4 and the endoscope operation part 3.

Next, the ultrasonic observation part 71, the endoscope observation part 75, the water supply tank 12, the suction pump 13, and the endoscope operation part 3 among the components of the ultrasonic endoscope 2 will be described in detail.

### [Ultrasonic Observation Part 71]

The ultrasonic observation part 71 is a portion that is provided to acquire an ultrasound image, and is disposed on the distal end side in the distal end portion 41 of the endoscope insertion part 4. As shown in Fig. 3, the ultrasonic observation part 71 comprises the ultrasonic transducer unit 74, a plurality of coaxial cables 82, and a flexible printed circuit (FPC) 83.

The ultrasonic transducer unit 74 corresponds to an ultrasound probe (probe), transmits ultrasonic waves using the ultrasonic transducer array 73, in which the plurality of ultrasonic transducers 72 described below are arranged, inside the body cavity of the patient, receives reflected waves (echoes) of the ultrasonic waves reflected by the observation target part, and outputs reception signals. The illustrated ultrasonic transducer unit 74 is a convex type, and transmits ultrasonic waves in a radial shape (arc shape). Note that the type (model) of the ultrasonic transducer unit 74 is not particularly limited, and other types may be used as long as ultrasonic waves can be transmitted and received, and for example, a linear type, a sector type, and a radial type, or the like may be used.

As shown in Fig. 3, the ultrasonic transducer unit 74 is configured by laminating a backing material layer 84, the ultrasonic transducer array 73, an acoustic matching layer 85, and an acoustic lens 86.

The ultrasonic transducer array 73 consists of the plurality of ultrasonic transducers 72 (ultrasonic transducers) arranged in a one-dimensional array. In more detail, the ultrasonic transducer array 73 is configured by arranging N (for example, N = 128) ultrasonic transducers 72 at regular intervals in a convex bent shape along an axial direction of the distal end portion 41 (a longitudinal axis direction of the endoscope insertion part 4). The ultrasonic transducer array 73 may be configured by arranging the plurality of ultrasonic transducers 72 in a two-dimensional array.

Each of the N ultrasonic transducers 72 is configured by disposing electrodes on both surfaces of a piezoelectric element (piezoelectric body). As the piezoelectric element, for example, a piezoelectric body consisting of piezoelectric ceramic represented by lead zirconate titanate (PZT), a polymer piezoelectric element represented by poly vinylidene di fluoride (PVDF), or piezoelectric single crystal represented by lead magnesium niobate-lead titanate (PMN-PT) can be used.

The electrodes consist of an individual electrode (not shown) individually provided for each of a plurality of ultrasonic transducers 72 and a transducer ground electrode (not shown) common to the plurality of ultrasonic transducers 72. In addition, these electrodes are electrically connected to the ultrasound image processor section 5 via the coaxial cable 82 and the FPC 83.

A pulsed drive voltage is supplied as an input signal (transmission signal) from the ultrasound image processor section 5 to each ultrasonic transducer 72 through the coaxial cable 82. In a case in which the drive voltage is applied to the electrodes of the ultrasonic transducer 72, the piezoelectric element expands and contracts to drive (vibrate) the ultrasonic transducer 72. As a result, pulsed ultrasonic waves are output from the ultrasonic transducer 72. In this case, the amplitude of the ultrasonic wave output from the ultrasonic transducer 72 has a magnitude corresponding to intensity (output intensity) in a case in which the ultrasonic transducer 72 outputs the ultrasonic wave. Here, the output intensity is defined as a magnitude of sound pressure of the ultrasonic wave output from the ultrasonic transducer 72.

Each ultrasonic transducer 72 vibrates (is driven) with reception of a reflected wave (echo) of the ultrasonic wave, and the piezoelectric element of each ultrasonic transducer 72 generates an electric signal. The electric signal is output as a reception signal of the ultrasonic wave from each ultrasonic transducer 72 toward the ultrasound image processor section 5. In this case, a magnitude (voltage value) of the electric signal output from the ultrasonic transducer 72 is a magnitude corresponding to reception sensitivity in a case in which the ultrasonic transducer 72 receives the ultrasonic wave. Here, the reception sensitivity is defined as a ratio of the amplitude of the electric signal output from the ultrasonic transducer 72 with reception of the ultrasonic wave to the amplitude of the ultrasonic wave transmitted from the ultrasonic transducer 72.

Then, as the N ultrasonic transducers 72 are sequentially driven by an electronic switch such as a transmission/reception control unit, scanning with ultrasonic waves is performed in a scanning range along a curved surface on which the ultrasonic transducer array 73 is disposed, for example, in a range of about several tens mm from the center of curvature of the curved surface. In more detail, in a case in which a brightness mode (B-mode) image is acquired as an ultrasound image, the drive voltage is supplied to m (for example, m = N/2) continuously arranged ultrasonic transducers 72 (hereinafter, referred to as drive target transducers) among the N ultrasonic transducers 72 by selecting an opening channel of the transmission/reception control unit. With this, the m drive target transducers are driven, and an ultrasonic wave is output from each drive target transducer of the opening channel. The ultrasonic waves output from the m drive target transducers are immediately composed, and the composite wave (ultrasound beam) is transmitted toward the observation target part. Thereafter, the m drive target transducers receive ultrasonic waves (echoes) reflected by the observation target part and output electric signals (reception signals) according to reception sensitivity at that time.

Then, the above-described series of steps (that is, the supply of the drive voltage, the transmission and reception of the ultrasonic waves, and the output of the electric signal) are repeatedly performed while shifting the positions of the drive target transducers in the N ultrasonic transducers 72 one by one (one ultrasonic transducer 72 at a time). Specifically, the above-described series of steps are started from the m drive target transducers on both sides of the ultrasonic transducer 72 located at one end among the N ultrasonic transducers 72. Then, the above-described series of steps are repeated each time the positions of the drive target transducers are shifted due to switching of the opening channel by the transmission/reception control unit. Finally, the above-described series of steps are repeatedly performed N times in total up to the m drive target transducers on both sides of the ultrasonic transducer 72 located at the other end among the N ultrasonic transducers 72.

The backing material layer 84 supports each ultrasonic transducer 72 of the ultrasonic transducer array 73 from a back surface side. The backing material layer 84 has a function of attenuating ultrasonic waves propagating to the backing material layer 84 side among ultrasonic waves emitted from the ultrasonic transducers 72 or ultrasonic waves (echoes) reflected by the observation target part. A backing material is a material having rigidity, such as hard rubber, and an ultrasonic wave attenuation material (ferrite, ceramics, or the like) is added as necessary.

The acoustic matching layer 85 is superimposed on the ultrasonic transducer array 73, and is provided for acoustic impedance matching between the body of the patient and the ultrasonic transducer 72. Since the acoustic matching layer 85 is disposed, it is possible to increase a transmittance of the ultrasonic wave. As a material of the acoustic matching layer 85, various organic materials whose acoustic impedance values are closer to the acoustic impedance of the body of the patient than the piezoelectric element of the ultrasonic transducer 72 can be used. Specific examples of the material of the acoustic matching layer 85 include epoxy resin, silicon rubber, polyimide, and polyethylene.

The acoustic lens 86 superimposed on the acoustic matching layer 85 is provided to converge the ultrasonic waves emitted from the ultrasonic transducer array 73 toward the observation target part. The acoustic lens 86 is made of, for example, silicon-based resin (millable silicon rubber, liquid silicon rubber, or the like), butadiene-based resin, or polyurethane-based resin, and a powder of titanium oxide, alumina, silica, or the like is mixed as necessary.

The FPC 83 is electrically connected to the electrode of each ultrasonic transducer 72. Each of the plurality of coaxial cables 82 is wired to the FPC 83 at one end thereof and is electrically connected to the ultrasound image processor section 5 at the other end (a side opposite to the FPC 83 side).

### [Endoscope Observation Part 75]

The endoscope observation part 75 is a portion that is provided to acquire an endoscope image, and is disposed on the base end side with respect to the ultrasonic observation part 71 in the distal end portion 41 of the endoscope insertion part 4. As shown in Figs. 2 and 3, the endoscope observation part 75 is configured of the observation window 77, the objective lens 78, the CCD 79, the illumination window 76, a cleaning nozzle 87, a wiring cable 88, and the like.

The observation window 77 is attached to the distal end portion 41 in a state of being obliquely inclined in the axial direction (the longitudinal axis direction of the endoscope insertion part 4) in the distal end portion 41 of the endoscope insertion part 4. The light reflected by the observation target adjacent part and incident from the observation window 77 is imaged on an imaging surface of the CCD 79 by the objective lens 78.

The CCD 79 photoelectrically converts reflected light of the observation target adjacent part transmitted through the observation window 77 and the objective lens 78 and formed on the imaging surface, and outputs an imaging signal. Instead of the CCD 79, a solid-state imaging element such as a complementary metal oxide semiconductor (CMOS) can also be used. The image signal output from the CCD 79 is transmitted to the endoscope image processor section 9 by the universal cable 6 by way of the wiring cable 88 extending from the endoscope insertion part 4 to the endoscope operation part 3.

The illumination windows 76 are provided at both side positions of the observation window 77. An emission end of the light guide (not shown) is connected to the illumination window 76. The light guide extends from the endoscope insertion part 4 to the endoscope operation part 3, and an incidence end of the light guide is connected to the light source device 8 connected via the universal cable 6. Illumination light emitted from the light source device 8 is transmitted through the light guide, and the observation target adjacent part is irradiated with the illumination light from the illumination windows 76.

The cleaning nozzle 87 is an ejection hole formed in the distal end portion 41 of the endoscope insertion part 4 to clean surfaces of the observation window 77 and the illumination window 76, and air or a cleaning liquid is ejected from the cleaning nozzle 87 toward the observation window 77 and the illumination window 76. The cleaning liquid ejected from the cleaning nozzle 87 can be water, particularly deaerated water. Note that the cleaning liquid is not particularly limited, and other liquids, for example, normal water (water that is not deaerated) may be used.

### [Water Supply Tank 12 and Suction Pump 13]

The water supply tank 12 is a tank that stores deaerated water, and is connected to the universal connector 7A by an air/water supply tube 12A. The deaerated water is used as the cleaning liquid that is ejected from the cleaning nozzle 87.

The suction pump 13 sucks aspirates (including deaerated water supplied for cleaning) into the body cavity through the treatment tool outlet port 80. The suction pump 13 is connected to the universal connector 7A by a suction tube 13A. The ultrasonic endoscope system 1 may comprise an air supply pump that supplies air to a predetermined air supply destination, or the like.

The treatment tool channel 81 and an air/water supply pipe line (not shown) are provided in the endoscope insertion part 4 and the endoscope operation part 3.

A treatment tool insertion port (forceps port) 31 disposed in the endoscope operation part 3 communicates with the treatment tool outlet port 80 through the treatment tool channel 81. In addition, the treatment tool channel 81 is connected to a suction button 32 disposed in the endoscope operation part 3. The suction button 32 is connected to the suction pump 13 in addition to the treatment tool channel 81.

The air/water supply pipe line communicates with the cleaning nozzle 87 on one end side, and is connected to an air/water supply button 33 provided in the endoscope operation part 3 on the other end side. The air/water supply button 33 is connected to the water supply tank 12 in addition to the air/water supply pipe line.

### [Endoscope Operation Part 3]

The endoscope operation part 3 is a portion that is operated by the user at the start of the ultrasound diagnosis, during the diagnosis, at the end of the diagnosis, and the like, to which one end of the universal cable 6 is connected. In addition, as shown in Fig. 1, the endoscope operation part 3 has the air/water supply button 33, the suction button 32, a pair of angle knobs 34, and the treatment tool insertion port 31.

In a case in which each of the pair of angle knobs 34 is rotationally moved, the bendable portion 42 of the endoscope insertion part 4 is remotely operated to be bent and deformed. With the deformation operation, the distal end portion 41 of the endoscope insertion part 4, in which the ultrasonic observation part 71 and the endoscope observation part 75 are disposed, can be directed to a desired direction.

The treatment tool insertion port 31 is a hole formed such that the treatment tool (not shown) such as forceps is inserted thereinto, and communicates with the treatment tool outlet port 80 via the treatment tool channel 81. The treatment tool inserted into the treatment tool insertion port 31 passes through the treatment tool channel 81 and is then introduced into the body cavity from the treatment tool outlet port 80.

The air/water supply button 33 and the suction button 32 are two-stage switching type push buttons, and are operated to switch opening and closing of the pipe line provided inside each of the endoscope insertion part 4 and the endoscope operation part 3.

### [Ultrasound Image Processor Section 5 and Endoscope Image Processor Section 9]

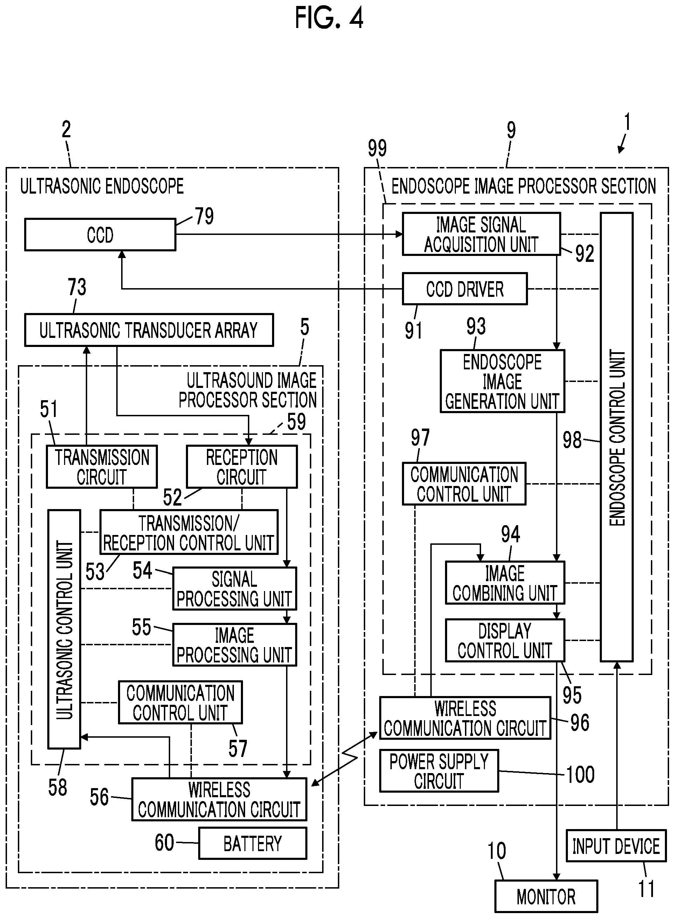

Fig. 4 shows configurations of the ultrasound image processor section 5 and the endoscope image processor section 9.

The ultrasound image processor section 5 has a transmission circuit 51 and a reception circuit 52 that are connected to the ultrasonic transducer array 73 disposed in the distal end portion 41 of the endoscope insertion part 4 of the ultrasonic endoscope 2. A transmission/reception control unit 53 is connected to the transmission circuit 51 and the reception circuit 52, a signal processing unit 54, an image processing unit 55, and a wireless communication circuit 56 are sequentially connected in series to the reception circuit 52, and a communication control unit 57 is connected to the wireless communication circuit 56.

An ultrasonic control unit 58 is connected to the transmission/reception control unit 53, the signal processing unit 54, the image processing unit 55, and the communication control unit 57.

The transmission circuit 51, the reception circuit 52, the transmission/reception control unit 53, the signal processing unit 54, the image processing unit 55, the communication control unit 57, and the ultrasonic control unit 58 constitute an ultrasonic processor 59.

In addition, a battery 60 is built in the ultrasound image processor section 5.

The transmission circuit 51 includes, for example, a plurality of pulse generators, adjusts a delay amount of each drive voltage based on a transmission delay pattern selected in accordance with a control signal from the transmission/reception control unit 53 so that the ultrasonic waves transmitted from the plurality of ultrasonic transducers 72 of the ultrasonic transducer array 73 form an ultrasound beam, and supplies each drive voltage to the plurality of ultrasonic transducers 72.

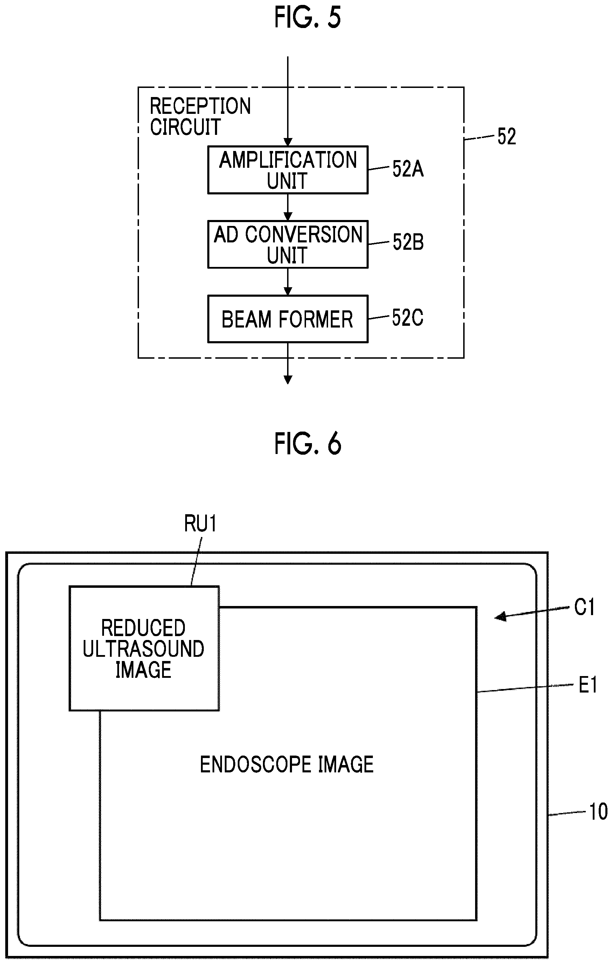

The reception circuit 52 processes the reception signals output from the ultrasonic transducer array 73 in accordance with a control signal from the transmission/reception control unit 53 to generate a sound ray signal. As shown in Fig. 5, the reception circuit 52 has a configuration in which an amplification unit 52A, an analog digital (AD) conversion unit 52B, and a beam former 52C are connected in series.

The amplification unit 52A amplifies the reception signal that is an analog signal input from each ultrasonic transducer 72 constituting the ultrasonic transducer array 73, and transmits the amplified reception signal to the AD conversion unit 52B.

The AD conversion unit 52B converts the analog reception signal transmitted from the amplification unit 52Ainto a digital signal to acquire reception data and sends the reception data to the beam former 52C.

The beam former 52C performs so-called reception focus processing of performing addition (phase addition) by applying a delay to each reception data following a set sound velocity based on a reception delay pattern selected in accordance with a control signal from the transmission/reception control unit 53. By performing this reception focus processing, a sound ray signal in which the focus of the ultrasound echo is narrowed down is generated.

The transmission/reception control unit 53 controls the transmission circuit 51 and the reception circuit 52 to transmit the ultrasound beam and receive the ultrasound echo based on an ultrasound image generation mode and a scanning method as instructed by the ultrasonic control unit 58. Here, the ultrasound image generation mode includes available examination modes in an ultrasound diagnostic apparatus, such as a brightness mode (B-mode), a motion mode (M-mode), an amplitude mode (A-mode), a color flow mode (CF-mode), a pulsed wave doppler mode (PW-mode), and a continuous wave doppler mode (CW-mode), and the scanning method indicates, for example, any one of an electronic sector scanning method, an electronic linear scanning method, an electronic convex scanning method, or the like.

The signal processing unit 54 performs envelope detection processing after performing, with respect to the sound ray signal generated by the reception circuit 52, correction of attenuation due to a propagation distance according to the depth of the position at which the ultrasonic wave is reflected, and generates a signal that is tomographic image information related to a tissue in the subject.

The image processing unit 55 raster-converts the signal generated by the signal processing unit 54 into an image signal according to a normal television signal scanning method, performs various kinds of necessary image processing, such as brightness correction, gradation correction, sharpness correction, and color correction, on the image signal generated in this manner to generate ultrasound image data (hereinafter, simply referred to as an ultrasound image), and then sends the ultrasound image data as ultrasound image information data to the wireless communication circuit 56.

The wireless communication circuit 56 includes an antenna for performing transmission and reception of radio waves, and wirelessly transmits the ultrasound image information data to the endoscope image processor section 9 by modulating a carrier based on the ultrasound image information data generated by the image processing unit 55 to generate a transmission signal and by supplying the transmission signal to the antenna to transmit radio waves from the antenna. As the carrier modulation method, amplitude shift keying (ASK), phase shift keying (PSK), quadrature phase shift keying (QPSK), 16 quadrature amplitude modulation (16QAM), and the like are used.

It is desirable that the wireless communication circuit 56 wirelessly transmits the ultrasound image information data to the endoscope image processor section 9 through so-called serial communication in which data is sequentially transmitted at once in units of one bit on a transmission line. By adopting the serial communication, it is possible to transmit data with a small number of the transmission lines without the need for synchronization between the transmission lines.

The communication control unit 57 controls the wireless communication circuit 56 such that the ultrasound image information data is transmitted with transmission radio wave intensity set by the ultrasonic control unit 58.

The wireless communication circuit 56 and the communication control unit 57 constitute an ultrasound image information data transmission unit that transmits the ultrasound image information data to the endoscope image processor section 9.

The ultrasonic control unit 58 controls each unit of the ultrasound image processor section 5 based on a program or the like stored in advance.

The battery 60 supplies a power supply voltage to each circuit in the ultrasound image processor section 5.

The ultrasonic processor 59 having the transmission circuit 51, the reception circuit 52, the transmission/reception control unit 53, the signal processing unit 54, the image processing unit 55, the communication control unit 57, and the ultrasonic control unit 58 is configured by using, for example, an application specific integrated circuit (ASIC). Note that the ultrasonic processor 59 may be configured by using a field programmable gate array (FPGA), a digital signal processor (DSP), a graphics processing unit (GPU), or other integrated circuits (ICs).

In addition, the ultrasonic processor 59 can be configured of a central processing unit (CPU) that executes various programs and a control program for causing the CPU to perform various processes. In this case, the transmission circuit 51, the reception circuit 52, the transmission/reception control unit 53, the signal processing unit 54, the image processing unit 55, the communication control unit 57, and the ultrasonic control unit 58 of the ultrasonic processor 59 can also be configured by being partially or entirely integrated into one CPU or the like.

The ultrasound image processor section 5 having such a configuration is housed in the endoscope operation part 3 of the ultrasonic endoscope 2.

On the other hand, the endoscope image processor section 9 has a CCD driver 91 and an image signal acquisition unit 92 connected to the CCD 79 disposed in the distal end portion 41 of the endoscope insertion part 4 of the ultrasonic endoscope 2. An endoscope image generation unit 93 is connected to the image signal acquisition unit 92, and an image combining unit 94 and a display control unit 95 are sequentially connected in series to the endoscope image generation unit 93. A wireless communication circuit 96 is connected to the image combining unit 94, and a communication control unit 97 is connected to the wireless communication circuit 96.

An endoscope control unit 98 is connected to the CCD driver 91, the image signal acquisition unit 92, the endoscope image generation unit 93, the image combining unit 94, the display control unit 95, and the communication control unit 97.

The CCD driver 91, the image signal acquisition unit 92, the endoscope image generation unit 93, the image combining unit 94, the display control unit 95, the communication control unit 97, and the endoscope control unit 98 constitute an endoscopic processor 99.

In addition, the endoscope image processor section 9 has a power supply circuit 100.

The monitor 10 is connected to the display control unit 95, and the input device 11 is connected to the endoscope control unit 98.

The CCD driver 91 inputs a drive signal to the CCD 79 based on a command from the endoscope control unit 98, and controls a readout timing of an accumulated electric charge of the CCD 79, a shutter speed of an electronic shutter of the CCD 79, and the like.

The image signal acquisition unit 92 performs noise removal and amplification on an analog imaging signal output from the CCD 79, converts the imaging signal into a digital image signal, and then sends the image signal to the endoscope image generation unit 93.

The endoscope image generation unit 93 performs various kinds of image processing on the image signal digitized by the image signal acquisition unit 92 to generate an endoscope image.

The wireless communication circuit 96 is wirelessly connected to the wireless communication circuit 56 of the ultrasound image processor section 5, and receives the ultrasound image as the ultrasound image information data wirelessly transmitted from the wireless communication circuit 56.

More specifically, the wireless communication circuit 96 includes an antenna for performing transmission and reception of radio waves, and receives a transmission signal transmitted by the wireless communication circuit 56 of the ultrasound image processor section 5 via the antenna and demodulates the received transmission signal to send the ultrasound image to the image combining unit 94.

The communication control unit 97 controls the wireless communication circuit 96 such that the transmission signal transmitted from the wireless communication circuit 56 of the ultrasound image processor section 5 is received.

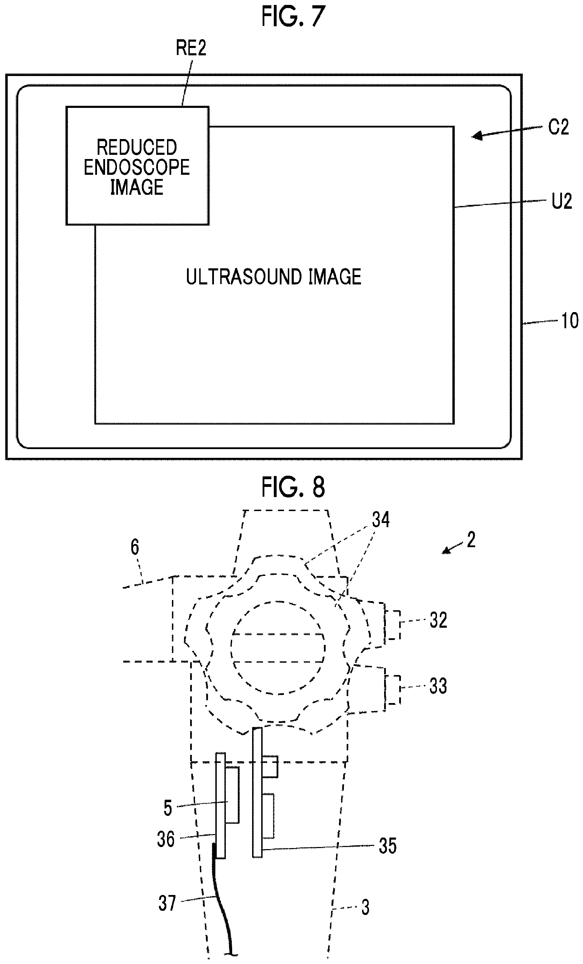

The image combining unit 94 generates a composite image in which the ultrasound image is combined with the endoscope image based on a command from the endoscope control unit 98. For example, as shown in Fig. 6, the image combining unit 94 generates a reduced ultrasound image RU1, which is reduced by reduction processing based on the ultrasound image sent from the wireless communication circuit 96, and generates a composite image C1 in which the reduced ultrasound image RU1 is superimposed on an endoscope image E1 generated by the endoscope image generation unit 93. Alternatively, as shown in Fig. 7, the image combining unit 94 generates a reduced endoscope image RE2, which is reduced by reduction processing based on the endoscope image generated by the endoscope image generation unit 93, and generates a composite image C2 in which the reduced endoscope image RE2 is superimposed on an ultrasound image U2 sent from the wireless communication circuit 96.

The display control unit 95 displays the composite image generated by the image combining unit 94 on the monitor 10 as a display image.

The endoscope control unit 98 controls each unit of the endoscope image processor section 9 based on a program stored in advance in a storage unit (not shown) or the like and an input operation performed by the user via the input device 11.

The power supply circuit 100 takes in power from, for example, a commercial power supply to generate an internal power supply voltage and supplies the internal power supply voltage to each circuit in the endoscope image processor section 9.

The endoscopic processor 99 having the CCD driver 91, the image signal acquisition unit 92, the endoscope image generation unit 93, the image combining unit 94, the display control unit 95, the communication control unit 97, and the endoscope control unit 98 is configured of a CPU and a control program for causing the CPU to perform various types of processing, but may be configured by using an FPGA, a DSP, an ASIC, a GPU, or other ICs, or may be configured by a combination thereof.

In addition, the CCD driver 91, the image signal acquisition unit 92, the endoscope image generation unit 93, the image combining unit 94, the display control unit 95, the communication control unit 97, and the endoscope control unit 98 of the endoscopic processor 99 can also be configured by being partially or entirely integrated into one CPU or the like.

As shown in Fig. 8, an operation member such as the suction button 32, the air/water supply button 33, or the pair of angle knobs 34 is disposed in the endoscope operation part 3 of the ultrasonic endoscope 2, and a control substrate 35 for controlling these operation members is housed in the endoscope operation part 3.

Similarly to the control substrate 35, the ultrasound image processor section 5 is housed in the endoscope operation part 3 of the ultrasonic endoscope 2 in a state of being mounted on the circuit board 36.

In addition, one end of a sheet-shaped heat transfer member 37 is fixed to the circuit board 36.

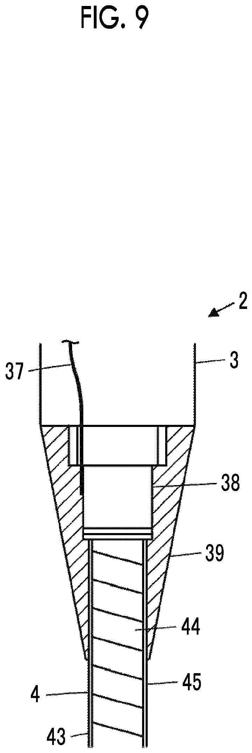

As shown in Fig. 9, a connection metal portion 38 for connecting a base end portion of the soft portion 43 of the endoscope insertion part 4 to the endoscope operation part 3 is disposed at an end portion of the endoscope operation part 3 of the ultrasonic endoscope 2 on the endoscope insertion part 4 side, and the other end of the sheet-shaped heat transfer member 37 is fixed to the connection metal portion 38. That is, the circuit board 36 on which the ultrasound image processor section 5 is mounted and the connection metal portion 38 are connected to each other via the heat transfer member 37. In addition, the soft portion 43 of the endoscope insertion part 4 has a configuration in which an outer peripheral portion of a spiral tube 44 around which a metal band-shaped member is helically wound is coated with an outer cover 45, and a base end portion of the spiral tube 44 is attached to the connection metal portion 38.

Therefore, the heat generated by the operation of the ultrasound image processor section 5 is configured to be transmitted to the spiral tube 44 of the soft portion 43 via the circuit board 36, the heat transfer member 37, and the connection metal portion 38, thereby promoting the heat radiation.

As a material of the heat transfer member 37, for example, a thermal sheet such as a carbon sheet, a material having excellent thermal conductivity, such as a copper sheet or an aluminum sheet having flexibility and a thin thickness, can be used. In addition, as a method of fixing the heat transfer member 37 to the circuit board 36 and the connection metal portion 38, various methods such as adhesion with an adhesive having excellent thermal conductivity, screwing, soldering, and press-fitting with an elastic member can be adopted.

A connecting portion between the endoscope operation part 3 and the endoscope insertion part 4 is covered with a cover 39 made of, for example, silicon rubber.

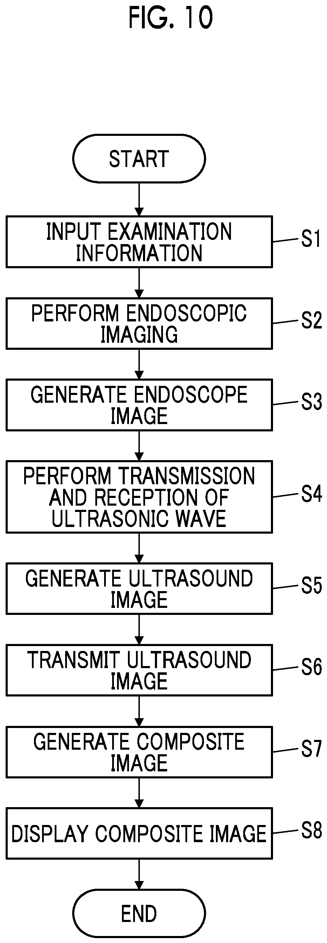

Next, the operation of the ultrasonic endoscope system 1 according to Embodiment 1 will be described with reference to the flowchart of Fig. 10.

In a case in which the power of each part of the ultrasonic endoscope system 1 is turned on in a state where the universal connector 7A is connected to the light source device 8 and the endoscope image processor section 9 in the ultrasonic endoscope 2, diagnosis processing using the ultrasonic endoscope system 1 is started using the turned-on power as a trigger.

In the diagnosis processing, first, in step S1, the user inputs examination information. The user can input examination information such as information on a patient who is a subject, an examination content, and various control parameters through the input device 11. In this case, the examination information related to the endoscope image processor section 9 is directly input from the input device 11 to the endoscope control unit 98 of the endoscope image processor section 9, while the examination information related to the ultrasound image processor section 5 is input from the input device 11 to the endoscope control unit 98 of the endoscope image processor section 9, and then wirelessly transmitted from the endoscope control unit 98 to the wireless communication circuit 56 of the ultrasound image processor section 5 through the communication control unit 97 and the wireless communication circuit 96, and input to the ultrasonic control unit 58.

Thereafter, in a case in which the user provides an instruction to start the diagnosis and the endoscope insertion part 4 of the ultrasonic endoscope 2 is inserted into the body cavity such as the digestive tract of the patient, endoscopic imaging is performed in step S2. That is, the drive signal is input to the CCD 79 by the CCD driver 91 based on a command from the endoscope control unit 98 of the endoscope image processor section 9, and the inside of the body cavity of the patient is imaged by the CCD 79.

The analog imaging signal output from the CCD 79 is transmitted from the ultrasonic endoscope 2 to the endoscope image processor section 9 via the universal cable 6 and the connection cable 7C, and is subjected to noise removal, amplification, and conversion into a digital image signal by the image signal acquisition unit 92.

In a case in which the digital image signal is sent from the image signal acquisition unit 92 to the endoscope image generation unit 93, in step S3, the endoscope image generation unit 93 performs various kinds of image processing on the digitized image signal to generate an endoscope image. The generated endoscope image is sent from the endoscope image generation unit 93 to the image combining unit 94.

In addition, in step S4, the transmission and reception of the ultrasonic waves are performed using the ultrasonic transducer array 73 disposed in the distal end portion 41 of the endoscope insertion part 4 of the ultrasonic endoscope 2. In this case, under the control of the transmission/reception control unit 53 of the ultrasound image processor section 5, the ultrasound beams from the plurality of ultrasonic transducers 72 of the ultrasonic transducer array 73 are transmitted from the inside of the body cavity of the patient toward the observation target part located on the outside of the body cavity in accordance with the drive signal from the transmission circuit 51. The ultrasound echo by the observation target part is received by the plurality of ultrasonic transducers 72 of the ultrasonic transducer array 73, and the reception signal, which is an analog signal, is output from the plurality of ultrasonic transducers 72 to the reception circuit 52.

The reception signal is amplified by the amplification unit 52A of the reception circuit 52, is subjected to AD conversion by the AD conversion unit 52B, and then is subjected to reception focus processing by the beam former 52C, whereby a sound ray signal is generated.

The sound ray signal is sent from the reception circuit 52 to the signal processing unit 54, and in step S5, the signal processing unit 54 and the image processing unit 55 generate an ultrasound image. That is, the signal processing unit 54 generates a signal, which is tomographic image information, by correcting attenuation caused by a distance according to the depth of the position where the ultrasonic waves are reflected and performing envelope detection processing, and the image processing unit 55 generates, for example, a B-mode ultrasound image by converting the signal into the image signal according to the normal television signal scanning method, and performing various kinds of necessary image processing such as gradation processing.

The ultrasound image generated in this way is transmitted from the ultrasound image processor section 5 to the endoscope image processor section 9 as the ultrasound image information data in step S6. In this case, the ultrasound image is wirelessly transmitted from the wireless communication circuit 56 of the ultrasound image processor section 5 housed in the endoscope operation part 3 of the ultrasonic endoscope 2 to the wireless communication circuit 96 of the endoscope image processor section 9.

The ultrasound image received by the wireless communication circuit 96 of the endoscope image processor section 9 is transmitted from the wireless communication circuit 96 to the image combining unit 94, and in step S7, the image combining unit 94 generates a composite image.

As shown in Fig. 6, for example, the image combining unit 94 generates the reduced ultrasound image RU1 by performing reduction processing on the ultrasound image sent from the wireless communication circuit 96, and generates the composite image C1 in which the reduced ultrasound image RU1 is superimposed on the endoscope image E1 generated by the endoscope image generation unit 93. Alternatively, as shown in Fig. 7, the reduced endoscope image RE2 is generated by performing reduction processing on the endoscope image generated by the endoscope image generation unit 93, and the composite image C2 in which the reduced endoscope image RE2 is superimposed on the ultrasound image U2 sent from the wireless communication circuit 96 is generated.

The composite image C1 or C2 generated by the image combining unit 94 is displayed on the monitor 10 by the display control unit 95 in step S8.

Instead of displaying the composite image C1 or C2 generated by the image combining unit 94 on the monitor 10, the endoscope image E1 and the ultrasound image U2 can each be displayed on the monitor 10 while switching between the endoscope image E1 and the ultrasound image U2, and the endoscope image E1 and the ultrasound image U2 can be simultaneously displayed side by side on the monitor 10.

As described above, in the ultrasonic endoscope system 1 according to Embodiment 1, the ultrasound image is generated based on the reception signal output from the ultrasonic transducer array 73 by the ultrasound image processor section 5 housed in the endoscope operation part 3 of the ultrasonic endoscope 2, and the generated ultrasound image is wirelessly transmitted from the wireless communication circuit 56 of the ultrasound image processor section 5 to the endoscope image processor section 9. Therefore, it is not necessary to dispose a dedicated ultrasound image processor device for generating an ultrasound image required in a normal ultrasonic endoscope system, outside the ultrasonic endoscope 2. The ultrasound image transmitted from the ultrasound image processor section 5 housed in the endoscope operation part 3 is two-dimensional image data similar to the endoscope image and can be processed by a normal endoscope image processor section. Accordingly, a small-sized ultrasonic endoscope system 1 that does not require any units other than the normal endoscope image processor section and has a simple configuration requiring only the preparation of the ultrasonic endoscope is realized.

As in JP2021-13453A described above, in a case in which the plurality of reception signals acquired by the plurality of ultrasonic transducers of the ultrasonic transducer array are transmitted from the ultrasonic endoscope to the ultrasound image processor device through the universal cable and the ultrasound image is generated by the ultrasound image processor device, a large number of signal lines corresponding to the plurality of ultrasonic transducers of the ultrasonic transducer array are required. Therefore, a diameter of the universal cable connected to the ultrasonic endoscope is increased, and there is a concern that the operability of the ultrasonic endoscope deteriorates.

However, in the ultrasonic endoscope system 1 according to Embodiment 1, the ultrasound image is generated by the ultrasound image processor section 5 housed in the endoscope operation part 3 of the ultrasonic endoscope 2, and the ultrasound image is transmitted from the ultrasound image processor section 5 to the endoscope image processor section 9 disposed outside the ultrasonic endoscope 2. Therefore, a diameter of the universal cable 6 connected to the ultrasonic endoscope 2 can be reduced, and the operability of the ultrasonic endoscope 2 is improved.

In addition, as in JP5815160B described above, in a case in which the plurality of reception signals acquired by the plurality of ultrasonic transducers of the ultrasonic transducer array are transmitted from the wireless communication unit of the ultrasonic endoscope to the ultrasonic observation device outside the ultrasonic endoscope, a transmission data amount increases and a load of wireless transmission increases, and a rate of the wireless transmission decreases, so that there is a concern that it is difficult to smoothly display the ultrasound image. In addition, a dedicated circuit for generating an ultrasound image from the plurality of reception signals is required, and the ultrasound image cannot be processed by a normal endoscopic processor section, so that a dedicated ultrasonic processor section is required.

However, in the ultrasonic endoscope system 1 according to Embodiment 1, the ultrasound image generated by the ultrasound image processor section 5 is wirelessly transmitted to the endoscope image processor section 9, instead of the plurality of reception signals acquired by the plurality of ultrasonic transducers 72 of the ultrasonic transducer array 73. Therefore, the load of the wireless transmission is reduced, and the ultrasound image can be smoothly displayed. In addition, a small-sized ultrasonic endoscope system 1 that does not require any units other than the normal endoscope image processor and has a simple configuration requiring only the preparation of the ultrasonic endoscope can be realized.

Since the ultrasound image is generated by the ultrasound image processor section 5 housed in the endoscope operation part 3 of the ultrasonic endoscope 2, heat generation occurs from the ultrasound image processor section 5, and the circuit board 36 on which the ultrasound image processor section 5 is mounted and the connection metal portion 38 that is disposed in the endoscope operation part 3 and is used for connecting the base end portion of the soft portion 43 of the endoscope insertion part 4 are connected to each other via the heat transfer member 37. Therefore, the heat generated in the ultrasound image processor section 5 is efficiently transmitted to the connection metal portion 38 and further transmitted from the connection metal portion 38 to the spiral tube 44 of the soft portion 43 of the endoscope insertion part 4, so that a temperature increase in the endoscope operation part 3 can be suppressed.

In Embodiment 1 described above, the B-mode ultrasound image is generated in step S5 of Fig. 10, but the present disclosure is not limited to the B mode. Similarly, the ultrasound image processor section 5 can also generate ultrasound images of an M-mode, an A-mode, a CF-mode, a PW-mode, and a CW-mode.

In addition, in Embodiment 1, the battery 60 built in the ultrasound image processor section 5 supplies the power supply voltage to each circuit in the ultrasound image processor section 5, but instead of the battery 60, for example, a power supply circuit that generates an internal power supply voltage by taking in power from a commercial power supply can also be disposed in the ultrasound image processor section 5, similarly to the power supply circuit 100 of the endoscope image processor section 9. Note that, in consideration of the fact that there is a need to pass a power supply line for connecting a commercial power supply to the power supply circuit of the ultrasound image processor section 5 through the universal cable 6 and the amount of heat generated by the power supply circuit, it is preferable that the battery 60 is built in the ultrasound image processor section 5.

In Embodiment 1 described above, the ultrasound image generated in the ultrasound image processor section 5 is wirelessly transmitted from the ultrasound image processor section 5 to the endoscope image processor section 9, but the ultrasound image can also be transmitted from the ultrasound image processor section 5 to the endoscope image processor section 9 in a wired manner. In this case, the ultrasound image is transmitted from the ultrasound image processor section 5 housed in the endoscope operation part 3 of the ultrasonic endoscope 2 to the endoscope image processor section 9 via the universal cable 6 shown in Fig. 1, but the ultrasound image generated by the ultrasound image processor section 5 is transmitted to the endoscope image processor section 9, instead of the plurality of reception signals acquired by the plurality of ultrasonic transducers 72 of the ultrasonic transducer array 73, so that the number of the signal lines passing through the universal cable 6 is suppressed, and the deterioration of the operability of the ultrasonic endoscope 2 can be suppressed.

Furthermore, since the frame rate of the ultrasound image is usually about 10 to 30 Hz and the image size is also small, it is also possible to transmit the ultrasound image to the same transmission path as a CCD image (about 60 Hz) in the free time for the CCD image transfer. The transmitted ultrasound image need only be separated from the CCD image on the endoscopic processor side and processed as an ultrasound image. It is desirable that the image size of the ultrasound image is reduced by Joint photographic experts group (JPEG) compression or the like. In this case, it is possible to remove the need for the dedicated signal line for the ultrasound image.

### Embodiment 2

In Embodiment 1 described above, the ultrasound image generated by the signal processing unit 54 and the image processing unit 55 of the ultrasound image processor section 5 is wirelessly transmitted to the endoscope image processor section 9 as the ultrasound image information data, but the intermediate data before the ultrasound image is generated can also be wirelessly transmitted as the ultrasound image information data.

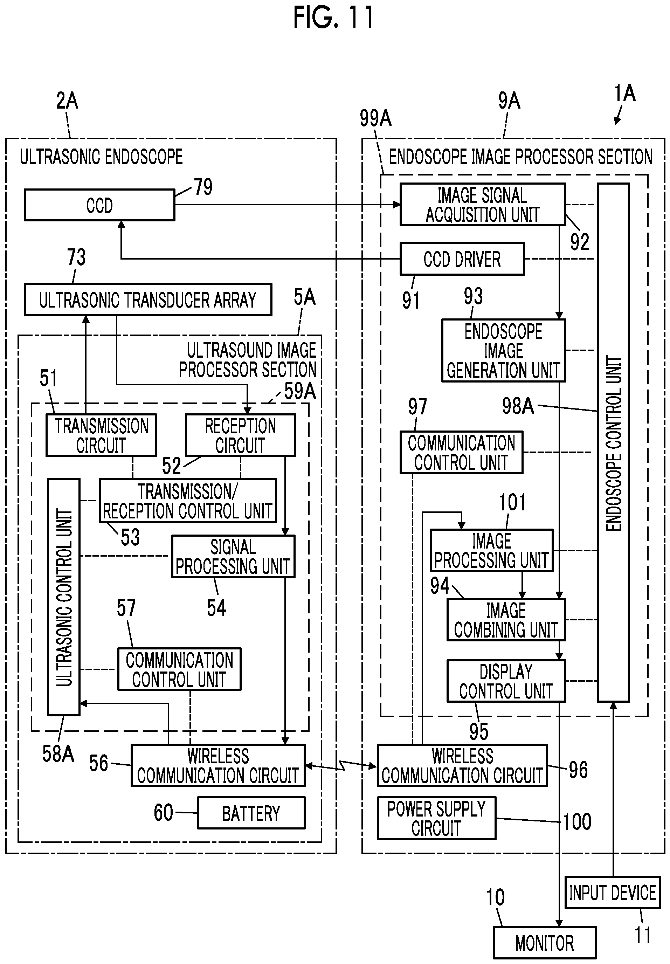

Fig. 11 shows a configuration of an ultrasonic endoscope system 1A according to Embodiment 2. The ultrasonic endoscope system 1A comprises an ultrasonic endoscope 2A instead of the ultrasonic endoscope 2 and comprises an endoscope image processor section 9A instead of the endoscope image processor section 9 in the ultrasonic endoscope system 1 of Embodiment 1 shown in Fig. 4.

The ultrasonic endoscope 2A has an ultrasound image processor section 5A instead of the ultrasound image processor section 5 in the ultrasonic endoscope 2 in Embodiment 1, and other components are the same as those in the ultrasonic endoscope 2 in Embodiment 1. The ultrasound image processor section 5A is the same as the ultrasound image processor section 5 in Embodiment 1 except that the image processing unit 55 is omitted, an ultrasonic control unit 58A is used instead of the ultrasonic control unit 58, and an ultrasonic processor 59A is used instead of the ultrasonic processor 59.

The transmission circuit 51, the reception circuit 52, the transmission/reception control unit 53, the signal processing unit 54, the communication control unit 57, and the ultrasonic control unit 58A constitute the ultrasonic processor 59A.

In addition, in the endoscope image processor section 9A, an image processing unit 101 is added, an endoscope control unit 98A is used instead of the endoscope control unit 98, and an endoscopic processor 99A is used instead of the endoscopic processor 99 in the endoscope image processor section 9 in Embodiment 1.

The CCD driver 91, the image signal acquisition unit 92, the endoscope image generation unit 93, the image combining unit 94, the display control unit 95, the communication control unit 97, the endoscope control unit 98A, and the image processing unit 101 constitute the endoscopic processor 99A.

In the ultrasound image processor section 5A, the signal processing unit 54 performs envelope detection processing after performing, with respect to the sound ray signal generated by the reception circuit 52, correction of attenuation due to a propagation distance according to the depth of the position at which the ultrasonic wave is reflected, and generates intermediate data that is tomographic image information related to a tissue in the subject.

The intermediate data is wirelessly transmitted from the wireless communication circuit 56 to the wireless communication circuit 96 of the endoscope image processor section 9A as the ultrasound image information data.

The image processing unit 101 added to the endoscope image processor section 9A is substantially the same as the image processing unit 55 of the ultrasound image processor section 5 in Embodiment 1, and generates an ultrasound image by performing raster conversion and various kinds of necessary image processing on the intermediate data received by the wireless communication circuit 96, and transmits the ultrasound image to the image combining unit 94.

In this way, even in a case in which the intermediate data before the ultrasound image is obtained is wirelessly transmitted from the ultrasound image processor section 5A to the endoscope image processor section 9A as the ultrasound image information data, the composite image C1 or C2 generated by the image combining unit 94 can be displayed on the monitor 10 as in Embodiment 1.

### Embodiment 3

In Embodiments 1 and 2 described above, the composite image C1 or C2 generated by the image combining unit 94 of the endoscope image processor section 9 or 9A is displayed on the monitor 10, but the present disclosure is not limited thereto, and the endoscope image and the ultrasound image can also be displayed on separate monitors, respectively.

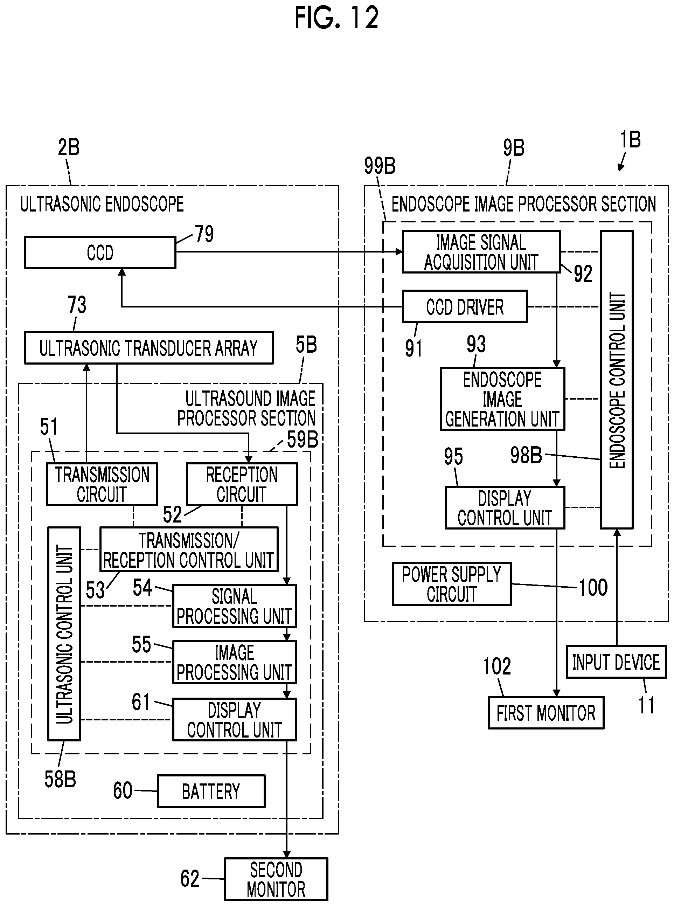

Fig. 12 shows a configuration of an ultrasonic endoscope system 1B according to Embodiment 3. The ultrasonic endoscope system 1B comprises an ultrasonic endoscope 2B instead of the ultrasonic endoscope 2, comprises an endoscope image processor section 9B instead of the endoscope image processor section 9, and comprises a first monitor 102 and a second monitor 62 instead of the monitor 10 in the ultrasonic endoscope system 1 of Embodiment 1 shown in Fig. 4. The first monitor 102 and the second monitor 62 constitute a display unit, each of which includes, for example, a display device such as an LCD and an organic EL display. The first monitor 102 and the second monitor 62 are disposed outside the ultrasonic endoscope 2B and the endoscope image processor section 9B.

The ultrasonic endoscope 2B has an ultrasound image processor section 5B instead of the ultrasound image processor section 5 in the ultrasonic endoscope 2 in Embodiment 1, and other components are the same as those in the ultrasonic endoscope 2 in Embodiment 1. The ultrasound image processor section 5B is the same as the ultrasound image processor section 5 in Embodiment 1 except that the wireless communication circuit 56 and the communication control unit 57 are omitted, a display control unit 61 is added, an ultrasonic control unit 58B is used instead of the ultrasonic control unit 58, and an ultrasonic processor 59B is used instead of the ultrasonic processor 59.

The display control unit 61 is connected to the image processing unit 55, and the second monitor 62 is connected to the display control unit 61.

The display control unit 61 displays the ultrasound image generated by the image processing unit 55 on the second monitor 62 as a display image.

The transmission circuit 51, the reception circuit 52, the transmission/reception control unit 53, the signal processing unit 54, the image processing unit 55, the ultrasonic control unit 58B, and the display control unit 61 constitute the ultrasonic processor 59B.

In addition, the endoscope image processor section 9B is the same as the endoscope image processor section 9 in Embodiment 1 except that the image combining unit 94, the wireless communication circuit 96, and the communication control unit 97 are omitted, an endoscope control unit 98B is used instead of the endoscope control unit 98, and an endoscopic processor 99B is used instead of the endoscopic processor 99.

The display control unit 95 is connected to the endoscope image generation unit 93, and the first monitor 102 is connected to the display control unit 95.

The display control unit 95 displays the endoscope image generated by the endoscope image generation unit 93 on the first monitor 102 as a display image.

The CCD driver 91, the image signal acquisition unit 92, the endoscope image generation unit 93, the display control unit 95, and the endoscope control unit 98B constitute the endoscopic processor 99B.

In the ultrasonic endoscope system 1B according to Embodiment 3, the ultrasound image processor section 5B does not transmit the ultrasound image information data to the endoscope image processor section 9B.

The display control unit 61 of the ultrasound image processor section 5B housed in the endoscope operation part 3 of the ultrasonic endoscope 2B is connected to the second monitor 62 via the universal cable 6 shown in Fig. 1, and the ultrasound image generated by the image processing unit 55 is displayed on the second monitor 62.

In addition, the endoscope image generated by the endoscope image generation unit 93 is displayed on the first monitor 102.

In this way, even in a case in which the endoscope image and the ultrasound image are separately displayed on the first monitor 102 and second monitor 62, the user can make a diagnosis by observing the display images of the first monitor 102 and the second monitor 62.

### Embodiment 4

In Embodiments 1 to 3 described above, the ultrasound image processor sections 5, 5A, and 5B have the battery 60 and the power supply voltage is supplied to each circuit in the ultrasound image processor sections 5, 5A, and 5B from the battery 60, but the present disclosure is not limited thereto.

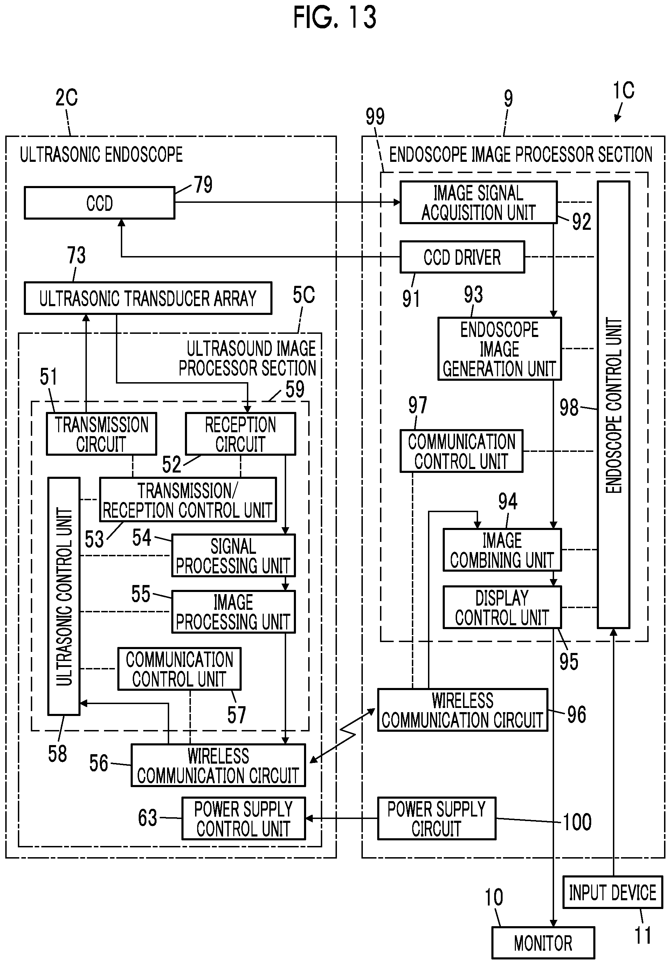

Fig. 13 shows a configuration of an ultrasonic endoscope system 1C according to Embodiment 4. The ultrasonic endoscope system 1C comprises an ultrasonic endoscope 2C instead of the ultrasonic endoscope 2 in the ultrasonic endoscope system 1 of Embodiment 1 shown in Fig. 4.

The ultrasonic endoscope 2C has an ultrasound image processor section 5C instead of the ultrasound image processor section 5 in the ultrasonic endoscope 2 in Embodiment 1, and other components are the same as those in the ultrasonic endoscope 2 in Embodiment 1. The ultrasound image processor section 5C has a power supply control unit 63 instead of the battery 60 in the ultrasound image processor section 5 in Embodiment 1, and other components are the same as those in the ultrasound image processor section 5 in Embodiment 1.

The power supply control unit 63 is connected to the power supply circuit 100 of the endoscope image processor section 9 via the universal cable 6 and the connection cable 7C shown in Fig. 1 from the ultrasound image processor section 5C housed in the endoscope operation part 3 of the ultrasonic endoscope 2. The power supply control unit 63 takes in the internal power supply voltage generated by the power supply circuit 100 of the endoscope image processor section 9 and supplies the internal power supply voltage to each circuit in the ultrasound image processor section 5C.

With this, the transmission circuit 51 can transmit the ultrasound beams from the plurality of ultrasonic transducers 72 by driving the ultrasonic transducer array 73 using the internal power supply voltage supplied from the power supply control unit 63, and the reception circuit 52, the signal processing unit 54, and the image processing unit 55 can generate the ultrasound image as the ultrasound image information data by processing the reception signals output from the ultrasonic transducer array 73 using the internal power supply voltage supplied from the power supply control unit 63.

Accordingly, even in a case in which the ultrasonic endoscope 2C does not have the battery 60, the ultrasound image processor section 5C housed in the endoscope operation part 3 can be driven by using the internal power supply voltage generated by the power supply circuit 100 of the endoscope image processor section 9. Therefore, the temperature increase in the endoscope operation part 3 due to the heat generation from the battery 60 is avoided, and the effort of checking the residual power of the battery 60, charging or replacing the battery 60, and the like can be saved.

### Embodiment 5

In Embodiments 1, 2, and 4 described above, the wireless communication circuit 96 disposed in the endoscope image processor section 9 or 9A receives the ultrasound image information data wirelessly transmitted from the ultrasound image processor section 5, 5A, or 5C housed in the endoscope operation part 3, but the wireless communication circuit 96 does not necessarily have to be disposed in the endoscope image processor section 9 or 9A.

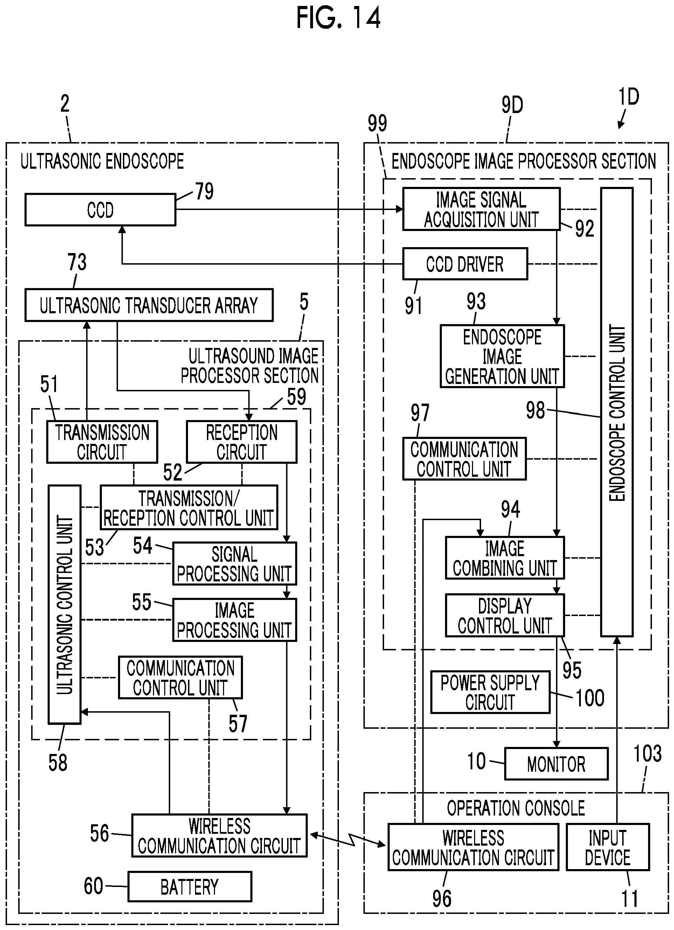

Fig. 14 shows a configuration of an ultrasonic endoscope system 1D according to Embodiment 5. The ultrasonic endoscope system 1D comprises an endoscope image processor section 9D instead of the endoscope image processor section 9 in the ultrasonic endoscope system 1 of Embodiment 1 shown in Fig. 4, in which an operation console 103 including the input device 11 is connected to the endoscope image processor section 9D.

The endoscope image processor section 9D is the same as the endoscope image processor section 9 in Embodiment 1 except that the wireless communication circuit 96 is omitted, and other components are the same as those in the endoscope image processor section 9 in Embodiment 1.

In addition, the operation console 103 includes the input device 11 and the wireless communication circuit 96, the input device 11 is connected to the endoscope control unit 98 of the endoscope image processor section 9D, and the wireless communication circuit 96 is connected to the image combining unit 94 and the communication control unit 97 of the endoscope image processor section 9D.

The wireless communication circuit 96 of the operation console 103 is wirelessly connected to the wireless communication circuit 56 of the ultrasound image processor section 5 housed in the endoscope operation part 3 of the ultrasonic endoscope 2, and receives a transmission signal wirelessly transmitted from the wireless communication circuit 56 of the ultrasound image processor section 5 and demodulates the received transmission signal under the control of the communication control unit 97 of the endoscope image processor section 9D to send the ultrasound image to the image combining unit 94 of the endoscope image processor section 9D.

Accordingly, even in a case in which the wireless communication circuit 96 is disposed on the operation console 103, as in Embodiment 1, the composite image C1 or C2 can be generated by the image combining unit 94 of the endoscope image processor section 9D and displayed on the monitor 10.

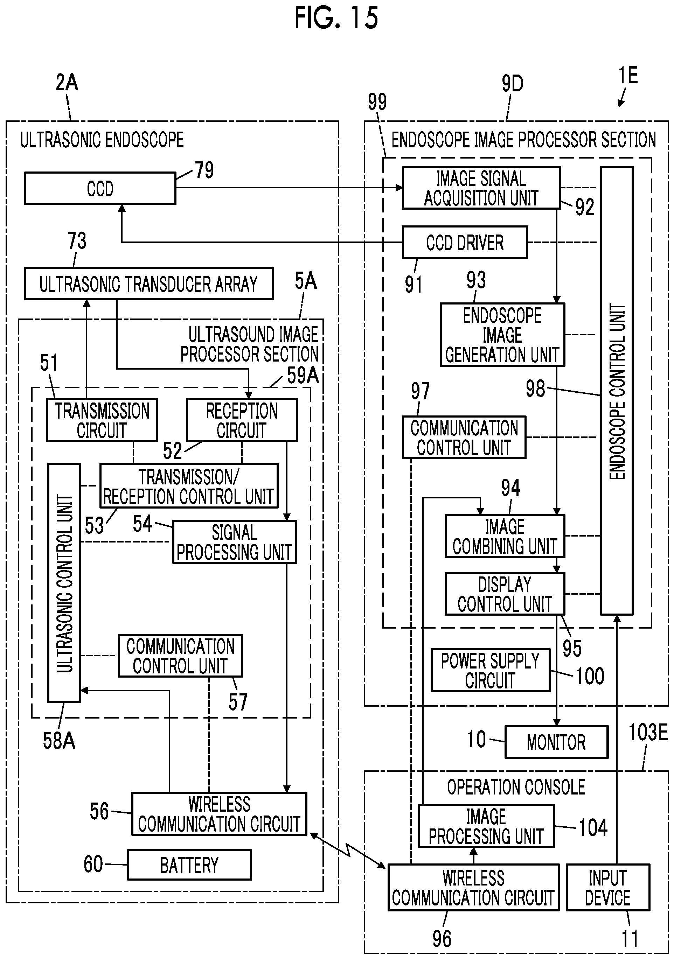

Furthermore, as in an ultrasonic endoscope system 1E shown in Fig. 15, the ultrasonic endoscope 2A used in Embodiment 2 can be provided instead of the ultrasonic endoscope 2, and the image processing unit 104 can be disposed on an operation console 103E. The ultrasonic processor 59A of the ultrasound image processor section 5A of the ultrasonic endoscope 2A does not have an image processing unit, and the signal processing unit 54 is connected to the wireless communication circuit 56.

The intermediate data before the ultrasound image is generated is wirelessly transmitted from the ultrasound image processor section 5A to the operation console 103E as the ultrasound image information data, the ultrasound image is generated in the image processing unit 104, and the ultrasound image is sent to the image combining unit 94 of the endoscope image processor section 9D. With this, even in a case of outputting the intermediate data, the endoscope image processor section 9D only needs to execute simple processing on the image as in Embodiment 1.

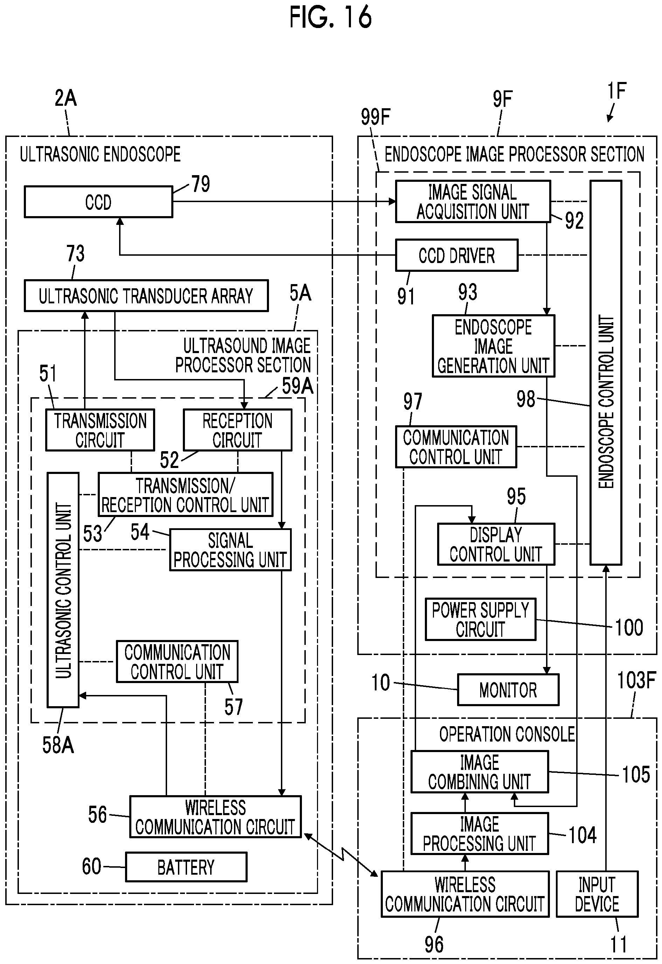

In addition, as in an ultrasonic endoscope system 1F shown in Fig. 16, an endoscope image processor section 9F can be provided instead of the endoscope image processor section 9D, and the image processing unit 104 and an image combining unit 105 can be disposed on an operation console 103F. An endoscopic processor 99F of the endoscope image processor section 9F does not have an image combining unit, and the image combining unit 105 of the operation console 103F of the endoscopic processor 99F is connected to the endoscope image generation unit 93 and the display control unit 95 of the endoscopic processor 99F.

In this case, a simple ultrasonic endoscope system iF can be configured by disposing the operation console 103F newly having these functions even in the endoscope image processor section having a hardware configuration in which the image combination and the display processing cannot be performed.

### Embodiment 6

In Embodiment 1 described above, as shown in Fig. 8, in the endoscope operation part 3 of the ultrasonic endoscope 2, the control substrate 35 for controlling the operation member such as the suction button 32, the air/water supply button 33, and the pair of angle knobs 34 and the circuit board 36 on which the ultrasound image processor section 5 is mounted are housed, and one end of the sheet-shaped heat transfer member 37 is fixed to the circuit board 36, but the present disclosure is not limited thereto.

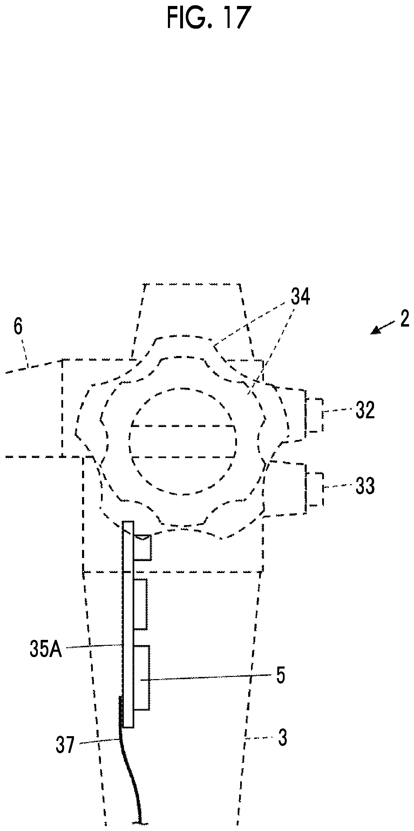

For example, as shown in Fig. 17, the ultrasound image processor section 5 can be mounted on a control substrate 35A for controlling the operation member.

In this way, the ultrasound image processor section 5 is mounted on the control substrate 35Afor controlling the operation member without using the dedicated circuit board 36 for mounting the ultrasound image processor section 5, so that it is possible to reduce the occupied space of the control substrate 35 and the circuit board 36 in the endoscope operation part 3.

Furthermore, one end of the sheet-shaped heat transfer member 37 is fixed to the control substrate 35A, so that it is possible to efficiently release the heat generated in the ultrasound image processor section 5 from the endoscope operation part 3.

### Explanation of References

1, 1A, 1B, 1C, 1D, 1E, 1F: ultrasonic endoscope system
2, 2A, 2B, 2C: ultrasonic endoscope
3: endoscope operation part
4: endoscope insertion part
5, 5A, 5B, 5C: ultrasound image processor section
6: universal cable
7A: universal connector
7B: endoscope connector
7C: connection cable
8: light source device
9, 9A, 9B, 9D, 9F: endoscope image processor section
10: monitor
11: input device
12: water supply tank
12A: air/water supply tube
13: suction pump
13A: suction tube
31: treatment tool insertion port
32: suction button
33: air/water supply button
34: angle knob
35, 35A: control substrate
36: circuit board
37: heat transfer member
38: connection metal portion
39: cover
41: distal end portion
42: bendable portion
43: soft portion
44: spiral tube
45: outer cover
51: transmission circuit
52: reception circuit
52A: amplification unit
52B: AD conversion unit
52C: beam former
53: transmission/reception control unit
54: signal processing unit
55, 101, 104: image processing unit
56: wireless communication circuit
57: communication control unit
58, 58A, 58B: ultrasonic control unit
59, 59A, 59B: ultrasonic processor
60: battery
61, 95: display control unit
62: second monitor
63: power supply control unit
71: ultrasonic observation part
72: ultrasonic transducer
73: ultrasonic transducer array
74: ultrasonic transducer unit
75: endoscope observation part
76: illumination window
77: observation window
78: objective lens
79: CCD
80: treatment tool outlet port
81: treatment tool channel
82: coaxial cable
83: FPC
84: backing material layer
85: acoustic matching layer
86: acoustic lens
91: CCD driver
92: image signal acquisition unit
93: endoscope image generation unit
94, 105: image combining unit
96: wireless communication circuit
97: communication control unit
98, 98A, 98B: endoscope control unit
99, 99A, 99B: endoscopic processor
100: power supply circuit
102: first monitor
103, 103E, 103F: operation console
RU1: reduced ultrasound image
RE2: reduced endoscope image
E1: endoscope image
U2: ultrasound image
C1, C2: composite image

## Claims

1. An ultrasonic endoscope system comprising:
an endoscope operation part that is operated by a user;
an endoscope insertion part that is connected to the endoscope operation part and is to be inserted into a subject;
an ultrasonic transducer array that is disposed at a distal end of the endoscope insertion part;
an endoscope image processor section that generates an endoscope image based on an image signal acquired optically by the endoscope insertion part;
an ultrasound image processor section that is housed in the endoscope operation part and generates ultrasound image information data by performing signal processing on a reception signal output from the ultrasonic transducer array; and
a display unit that displays an ultrasound image based on the ultrasound image information data and the endoscope image.

2. The ultrasonic endoscope system according to claim 1,
wherein the ultrasound image processor section includes an ultrasound image information data transmission unit that transmits the ultrasound image information data to the endoscope image processor section.

3. The ultrasonic endoscope system according to claim 2,
wherein the ultrasound image information data transmission unit transmits the ultrasound image information data to the endoscope image processor section through serial communication.

4. The ultrasonic endoscope system according to claim 2,
wherein the ultrasound image information data transmission unit wirelessly transmits the ultrasound image information data to the endoscope image processor section.

5. The ultrasonic endoscope system according to claim 4, further comprising:
an input device that is connected to the endoscope image processor section and is used by the user to perform an input operation.

6. The ultrasonic endoscope system according to claim 5, further comprising:
an operation console on which the input device is disposed,
wherein the operation console has a wireless communication circuit that receives the ultrasound image information data wirelessly transmitted from the ultrasound image information data transmission unit and sends the ultrasound image information data to the endoscope image processor section.

7. The ultrasonic endoscope system according to any one of claims 2 to 6,
wherein the endoscope image processor section includes an image combining unit that generates a composite image in which the ultrasound image is combined with the endoscope image.

8. The ultrasonic endoscope system according to claim 7,
wherein the display unit consists of a monitor for displaying the composite image.

9. The ultrasonic endoscope system according to claim 1,
wherein the display unit includes a first monitor for displaying the endoscope image and a second monitor for displaying the ultrasound image.

10. The ultrasonic endoscope system according to any one of claims 2 to 6,
wherein the ultrasound image information data is intermediate data obtained by subjecting a sound ray signal generated by performing the signal processing on the reception signal to attenuation correction according to a depth of a position where an ultrasonic wave is reflected and detection processing.

11. The ultrasonic endoscope system according to any one of claims 2 to 6,
wherein the ultrasound image information data is ultrasound image data obtained by subjecting a sound ray signal generated by performing the signal processing on the reception signal to attenuation correction according to a depth of a position where an ultrasonic wave is reflected and detection processing and converting the sound ray signal according to a predetermined image display method.

12. The ultrasonic endoscope system according to any one of claims 2 to 6,
wherein the endoscope image processor section has a power supply circuit that generates an internal power supply voltage, and
the ultrasound image processor section drives the ultrasonic transducer array using the internal power supply voltage generated by the power supply circuit and generates the ultrasound image information data.

13. The ultrasonic endoscope system according to any one of claims 2 to 6,
wherein the endoscope operation part has an operation member and a control substrate for controlling the operation member, and
the ultrasound image processor section is mounted on the control substrate.

14. The ultrasonic endoscope system according to any one of claims 2 to 6,
wherein the endoscope operation part has a connection metal portion to be connected to the endoscope insertion part,
a heat transfer member that connects the ultrasound image processor section and the connection metal portion is provided, and
heat generated by the ultrasound image processor section is transmitted to the connection metal portion via the heat transfer member.

15. A method of operating an ultrasonic endoscope system, the method comprising:
imaging an inside of a subject with an endoscope insertion part connected to an endoscope operation part;
generating an endoscope image by an endoscope image processor section based on an image signal acquired optically by the endoscope insertion part;
transmitting and receiving an ultrasonic wave by using an ultrasonic transducer array disposed at a distal end of the endoscope insertion part;
generating ultrasound image information data for the inside of the subject by performing signal processing on a reception signal output from the ultrasonic transducer array by an ultrasound image processor section housed in the endoscope operation part; and
displaying an ultrasound image based on the ultrasound image information data and the endoscope image on a display unit.
